# EUROPEAN PATENT SPECIFICATION

(11) **EP 0 938 572 B1**
(45) Date of publication and mention of the grant of the patent: **29.09.2004**
(21) Application number: 97900916.4
(22) Date of filing: 31.01.1997
(51) Int. Cl.: C12N 15/82, A01H 5/00

(54) **PROMOTER FROM TOBACCO**
PROMOTOR AUS TABAK
PROMOTEUR PROVENANT DU TABAC

(30) Priority: 01.02.1996 CA 2168617; 01.02.1996 US 593121
(43) Date of publication of application: 01.09.1999
(73) Proprietor: Her Majesty The Queen in Right of Canada, as Represented by The Minister of Agriculture and Agri-Food, Ottawa, Ontario K1A OC6 (CA); CARLETON UNIVERSITY, Ottawa, Ontario K1S 5B6 (CA)
(72) Inventor: FOBERT, Pierre, Saskatoon, Saskatchewan S7J 4J7 (CA); IYER, Venkatram, N., Kelowna, British Columbia V1W 2R9 (CA); MIKI, Brian, Ottawa, Ontario K1H 5V1 (CA); Hattori, Jiro, Ottawa, Ontario K1G 1M5 (CA); LABBE, Hélène, Ottawa, Ontario K1Y 1B8 (CA); OUELLET, Thérèse, Nepean, Ontario K2J 3X7 (CA); JAMES, Elizabeth, Eva, Kinburn, Ontario K0A 2H0 (CA); FOSTER-ATKINSON, Elizabeth, Nepean, Ontario K2G 5B6 (CA)
(74) Representative: Luderschmidt, Schüler & Partner GbR
(86) International application number: PCT/CA1997/000064
(87) International publication number: WO 1997/028268

(56) References cited:
- EP-A- 0 342 926
- EP-A- 0 559 603
- WO-A-94/21793
- PLANT MOLECULAR BIOLOGY, vol. 17, 1991, pages 837-851, XP002030411 FOBERT, P.R., ET SL.: "Detection of gene regulatory signals in plants revealed by T-DNA-mediated fusions" cited in the application
- GENE, vol. 94, 1990, pages 155-163, XP002030414 GHEYSEN, G., ET AL.: "Cloning and sequence analysis of truncated T-DNA inserts from Nicotiana tabacum"
- C.R. ACAD. SCI. PARIS, SCIENCE DE LA VIE, vol. 318, 1995, pages 121-128, XP002023143 DEVIC, M., ET AL.: "Assessment of promoter trap as a tool to study zygotic embryogenesis in Arabidopsis thaliana"
- TRANSGENIC RESEARCH, vol. 2, no. 1, 1993, pages 33-47, XP002000606 LINDSEY K ET AL: "TAGGING GENOMIC SEQUENCES THAT DIRECT TRANSGENE EXPRESSION BY ACTIVATION OF A PROMOTER TRAP IN PLANTS" cited in the application
- THE PLANT JOURNAL , vol. 6, no. 4, 1994, pages 567-577, XP002030412 FOBERT, P.R., ET AL.: "T-DNA tagging of a seed coat-specific cryptic promoter in tobacco" cited in the application
- MOLECULAR AND GENERAL GENETICS, vol. 220, 1990, pages 425-432, XP002030418 SIEMEISTER, G., ET AL.: "Genes for the plastid elongation factor Tu and ribosomal protein S7 and six tRNA genes on the 73kb DNA from Astia longa that resembles the chloroplast DNA of Euglena"

## Description

### FIELD OF INVENTION

The present invention relates to a promoter identified from *Nicotiana tabacum* (tobacco). This invention further relates to the use of said promoter to control the expression of exogenous DNAs of interest in transgenic plants of diverse plant species.

### BACKGROUND OF THE INVENTION

Bacteria from the genus *Agrobacterium* have the ability to transfer specific segments of DNA (T-DNA) to plant cells, where they stably integrate into the nuclear chromosomes. Analyses of plants harbouring the T-DNA have revealed that this genetic element may be integrated at numerous locations, and can occasionally be found within genes. One strategy which has been exploited to identify integration events within genes is to transform plant cells with specially designed T-DNA vectors which contain a reporter gene, devoid of *cis*-acting transcriptional and translational expression signals (i.e. promoterless), located at the end of the T-DNA. Upon integration, the initiation codon of the promoterless gene (reporter gene) will be juxtaposed to plant sequences. The consequence of T-DNA insertion adjacent to, and downstream of, gene promoter elements may be the activation of reporter gene expression. The resulting hybrid genes, referred to as T-DNA-mediated gene fusions, consist of unknown and thus un-characterized plant promoters residing at their natural location within the chromosome, and the coding sequence of a marker gene located on the inserted T-DNA (Fobert *et al*., 1991, Plant Mol. Biol. **17,** 837-851).

It has generally been assumed that activation of promoterless or enhanceness marker genes result from T-DNA insertions within or immediately adjacent to genes. The recent isolation of several T-DNA insertional mutants (Koncz *et al*., 1992, *Plant Mol. Biol*. **20,** 963-976; reviewed in Feldmann, 1991, *Plant J.* **1**, 71-82; Van Lijsebettens *et al*., 1991, *Plant Sci.* **80**, 27-37; Walden *et al*., 1991, *Plant J.* **1**: 281-288; Yanofsky *et al*., 1990, *Nature* **346**, 35-39), shows that this is the case for at least some insertions. However, other possibilities exist. One of these possibilities is that integration of the T-DNA activates silent regulatory sequences that are not associated with genes. Lindsey *et al*. (1993, *Transgenic Res.* **2,** 33-47) referred to such sequences as "pseudo-promoters" and suggested that they may be responsible for activating marker genes in some transgenic lines. Fobert *et al*. (1994, *Plant J.* **6,** 567-577) have cloned such sequences and have referred to these as "cryptic promoters".

### SUMMARY OF THE INVENTION

The present invention is directed to a promoter identified from *Nicotiana tabacum* (tobacco).

The transgenic tobacco plant, T1275, contained a 4.38 kb *Eco*RI/*Xba*I fragment containing the 2.15 kb promoterless *GUS-nos* gene and 2.23 kb of 5' flanking tobacco DNA (2225 bp). This 5' flanking DNA showed no homology to known sequences.

Thus, this invention embraces a promoter characterized in that it comprises at least an 18 bp contiguous sequence of SEQ ID NO:1.

The promoter could not be detected in soybean, potato, sunflower, *Arabidopsis, B. napus, B. oleracea,* corn, wheat or black spruce by Southern blot analysis. Expression of the cloned fragment in transgenic tobacco, *N. tabacum* c.v. Petit Havana, SRI and transgenic *B. napus* c.v. Westar was observed in leaf, stem, root, developing seed and flower. By transient expression analysis, *GUS* activity was also observed in leaf tissue of soybean, alfalfa, *Arabidopsis,* tobacco, *B. napus,* pea and suspension cultured cells of oat, com, wheat and barley.

Thus this invention also provides for a promoter that is a constitutive promoter. Furthermore, this promoter functions in diverse plant species when introduced on a cloning vector.

The present invention also embraces a constitutive promoter having a DNA sequence, substantially homologous to SEQ ID NO: 1.

The transcription start site for the introduced *GUS* gene in transgenic tobacco was located in the plant DNA upstream of the insertion site. It was the same in leaf, stem, root, seeds and flower. Furthermore, the native site was silent in both untransformed and transgenic tobacco.

Thus according to the present invention there is provided a constitutive promoter from tobacco that is cryptic, and that functions in diverse plant species when introduced in a cloning vector.

This invention also relates to a chimeric gene construct comprising a DNA of interest for which constitutive expression is desired, and a constitutive promoter, comprising at least an 18 bp contiguous sequence of SEQ ID NO: 1.

This invention further relates to a cloning vector containing said chimeric gene construct.

This invention also includes a plant cell which has been transformed with said chimeric gene, or said cloning vector. Furthermore, this invention embraces transgenic plants containing said chimeric gene, or said cloning vector.

This invention further relates to any transgenic plant containing a constitutive promoter, having a DNA sequence substantially homologous to SEQ ID NO: 1, operatively linked to a DNA region that is transcribed into RNA.

Also included in the present invention is a method of conferring constitutive expression of a gene in a plant, comprising: operatively linking an exogenous DNA of interest, for which constitutive expression is desired, with a constitutive promoter comprising at least an 18 bp contiguous sequence of SEQ ID NO:1, to produce a chimeric gene construct and introducing the chimeric gene construct into a plant capable of expressing the chimeric gene construct.

### BRIEF DESCRIPTION OF THE DRAWINGS

These and other features of the invention will become more apparent from the following description in which reference is made to the appended drawings wherein:
**FIGURE 1** shows the constitutive expression of *GUS* in all tissues of plant T1275, including leaf segments (a), stem cross-sections (b), roots (c), flower cross-sections (d), ovary cross-sections (e), immature embryos (f), mature embryos (g), and seed cross-sections (h).
**FIGURE 2** shows the *GUS* specific activity, which reveals that the level of *GUS* expression in T1275 is comparable to levels in plants expressing CaMV 35S - *GUS - nos* genes in leaf tissues.
**FIGURE 3** is the Southern blot analysis of *Eco* RI digested T1275 DNA with a *GUS* gene coding region probe (lane 1) and a *nptII* gene coding region probe (lane 2) revealing a single T-DNA insertion site in plant T1275.
**FIGURE 4** shows the cloned *GUS* gene fusion from pT1275. The arrow indicates the *GUS* mRNA start site within the plant DNA sequence.
**FIGURE 5** shows the restriction map of the isolated plant DNA sequence. The arrow indicates the *GUS* mRNA start site within the plant DNA sequence.
**FIGURE 6** shows that *GUS* specific activity varies in leaves of individual, regenerated, greenhouse-grown tobacco plants selected at random (Figure 6A) and is generally correlated with the level of accumulated *GUS* mRNA measured by RNase protection assay and densitometry of autoradiograms (Figure 6B).
**FIGURE 7** shows that transcripts corresponding to the native plant sequence *in situ* do not accumulate (3) in leaves of untransformed (lane U) or transgenic (lane T25) tobacco plants; whereas, transcripts corresponding to the same plant sequence fused to the *GUS* gene within the T-DNA inserted into transgenic plant T25 (lane T25) are present in the protected fragment indicated (2). The undigested probe (1) is presented in lane P as a control.

### DESCRIPTION OF PREFERRED EMBODIMENT

The present invention relates to plant gene regulatory sequences. Specifically this invention relates to a promoter, identified by T-DNA tagging with a promoterless β-glucuronidase gene (*GUS*) to generate a transgenic *N. tabacum* plant that expresses *GUS* activity constitutively.

This invention is also directed to a promoter that comprises a nucleotide sequence of at least 18 contiguous base pairs of SEQ ID NO:1. Oligonucleotides of 18 bp or more are useful in constructing heterologous promoters that comprise fragments of the promoter as defined in SEQ ID NO:1. The use of such heterologous promoters is well established in the literature. For example, fragments of specific elements within the 35S CaMV promoter have been duplicated or combined with other promoter fragments to produce chimeric promoters with desired properties (e.g. U.S. 5,491,288, 5,424,200, 5,322,938, 5,196,525, 5,164,316). Furthermore, oligonucleotides of 18 bps or longer are useful as probes or PCR primers in identifying or amplifying related DNA or RNA sequences in other tissues or organisms.

In the context of this disclosure, the term "promoter" or "promoter region" refers to a sequence of DNA, usually upstream (5') to the coding sequence of a structural gene, which controls the expression of the coding region by providing the recognition for RNA polymerase and/or other factors required for transcription to start at a particular site.

There are generally two types of promoters, inducible and constitutive promoters. An inducible promoter is a promoter that is capable of directly or indirectly activating transcription of one or more DNA sequences or genes in response to an inducer. In the absence of an inducer the DNA sequences or genes will not be transcribed. Typically the protein factor, that binds specifically to an inducible promoter to activate transcription, is present in an inactive form which is then directly or indirectly converted to the active form by the inducer. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. A plant cell containing an inducible promoter may be exposed to an inducer by externally applying the inducer to the cell or plant such as by spraying, watering, heating or similar methods.

A constitutive promoter directs the expression of a gene throughout the various parts of a plant and continuously throughout plant development. Examples of known constitutive promoters include those associated with the CaMV 35S transcript. (Odell et al., 1985, *Nature,* **313:** 810-812), the rice actin 1 (Zhang et al, 1991, *Plant Cell,* **3:** 1155-1165) and triosephosphate isomerase 1 (Xu et al, 1994, *Plant Physiol*. **106**: 459-467) genes, the maize ubiquitin 1 gene (Cornejo et al, 1993, *Plant Mol. Biol.* **29:** 637-646), the *Arabidopsis* ubiquitin 1 and 6 genes (Holtorf et al, 1995, *Plant Mol. Biol*. **29:** 637-646), and the tobacco translational initiation factor 4A gene (Mandel et al, 1995 *Plant Mol. Biol.* **29:** 995-1004). The present invention is directed to a DNA sequence which contains a promoter capable of directing the expression of a gene. Preferably the promoter is a constitutive promoter isolated from *N. tabacum.*

The term "constitutive" as used herein does not necessarily indicate that a gene is expressed at the same level in all cell types, but that the gene is expressed in a wide range of cell types, although some variation in abundance is often observed.

The present invention is further directed to a chimeric gene construct containing a DNA of interest operatively linked to the promoter of the present invention. Any exogenous gene can be used and manipulated according to the present invention to result in the expression of said exogenous gene. A DNA of interest may include, but is not limited to, a gene encoding a protein, a DNA that is transcribed to produce antisense RNA, or a transcript product that functions in some manner that mediates the expression of other DNAs, for example that results in the co-suppression of other DNAs or the like.

The chimeric gene construct of the present invention can further comprise a 3' untranslated region. A 3' untranslated region refers to that portion of a gene comprising a DNA segment that contains a polyadenylation signal and any other regulatory signals capable of effecting mRNA processing or gene expression. The polyadenylation signal is usually characterized by effecting the addition of polyadenylic acid tracks to the 3' end of the mRNA precursor. Polyadenylation signals are commonly recognized by the presence of homology to the canonical form 5' AATAAA-3' although variations are not uncommon.

Examples of suitable 3' regions are the 3' transcribed non-translated regions containing a polyadenylation signal of *Agrobacterium* tumor inducing (Ti) plasmid genes, such as the nopaline synthase (*Nos* gene) and plant genes such as the soybean storage protein genes and the small subunit of the ribulose-1, 5-bisphosphate carboxylase (ssRUBISCO) gene. The 3' untranslated region from the structural gene of the present construct can therefore be used to construct chimeric genes for expression in plants.

The chimeric gene construct of the present invention can also include further enhancers, either translation or transcription enhancers, as may be required. These enhancer regions are well known to persons skilled in the art, and can include the ATG initiation codon and adjacent sequences. The initiation codon must be in phase with the reading frame of the coding sequence to ensure translation of the entire sequence. The translation control signals and initiation codons can be from a variety of origins, both natural and synthetic. Translational initiation regions may be provided from the source of the transcriptional initiation region, or from the structural gene. The sequence can also be derived from the promoter selected to express the gene, and can be specifically modified so as to increase translation of the mRNA.

To aid in identification of transformed plant cells, the constructs of this invention may be further manipulated to include plant selectable markers. Useful selectable markers include enzymes which provide for resistance to an antibiotic such as gentamycin, hygromycin, kanamycin, and the like. Similarly, enzymes providing for production of a compound identifiable by colour change such as *GUS* (β-glucuronidase), or luminescence, such as luciferase are useful.

Also considered part of this invention are transgenic plants containing the chimeric gene construct comprising the promoter of the present invention. Methods of regenerating whole plants from plant cells are known in the art, and the method of obtaining transformed and regenerated plants is not critical to this invention. In general, transformed plant cells are cultured in an appropriate medium, which may contain selective agents such as antibiotics, where selectable markers are used to facilitate identification of transformed plant cells. Once callus forms, shoot formation can be encouraged by employing the appropriate plant hormones in accordance with known methods and the shoots transferred to rooting medium for regeneration of plants. The plants may then be used to establish repetitive generations, either from seeds or using vegetative propagation techniques.

The constructs of the present invention can be introduced into plant cells using Ti plasmids, Ri plasmids, plant virus vectors, direct DNA transformation, micro-injection, electroporation, etc. For reviews of such techniques see for example Weissbach and Weissbach, *Methods for Plant Molecular Biology*, Academy Press, New York VIII, pp. 421-463 (1988); and Geierson and Corey, *Plant Molecular Biology,* 2d Ed. (1988). The present invention further includes a suitable vector comprising the chimeric gene construct.

When specific sequences are referred to in the present invention, it is understood that these sequences include within their scope sequences that are "substantially homologous" to said specific sequences. Sequences are "substantially homologous" when at least about 70%, preferably at least about 80% and most preferably at least about 90% of the nucleotides match over a defined length of the molecule. Sequences that are "substantially homologous" include any substitution, deletion, or addition within the sequence. DNA sequences that are substantially homologous can be identified in Southern hybridization experiments, for example under stringent hybridization conditions (see Maniatis et al., in Molecular Cloning (A Laboratory Manual), Cold Spring Harbor Laboratory (1982) p 387 to 389).

The specific sequences, referred to in the present invention, also include sequences which are "functionally equivalent" to said specific sequences. In the present invention functionally equivalent sequences refer to sequences which although not identical to the specific sequences provide the same or substantially the same function. DNA sequences that are functionally equivalent include any substitution, deletion or addition within the sequence. With reference to the present invention functionally equivalent sequences will direct the expression of an exogenous gene constitutively.

While this invention is described in detail with particular reference to preferred embodiments thereof, said embodiments are offered to illustrate but not limit the invention.

### EXAMPLES

### Characterization of a Constitutive promoter - GUS Fusion

Transfer of binary constructs to *Agrobacterium* and leaf disc transformation of *N*. *tabacum* SR1 were performed as described by Fobert *et al*. (1991, *Plant Mol. Biol.* **17,** 837-851). Plant tissue was maintained on 100 µg/ml kanamycin sulfate (Sigma) throughout *in vitro* culture.

From the transgenic plants produced, one of these, T1275, was chosen for detailed study because of its high level and constitutive expression of *GUS.*

Fluorogenic and histological *GUS* assays were performed according to Jefferson (*Plant Mol. Biol. Rep.,* 1987, **5,** 387-405), as modified by Fobert *et al*. *(Plant Mol. Biol*., 1991, **17**, 837-851). For initial screening, leaves were harvested from *in vitro* grown plantlets. Later nine different tissues: leaf (L), stem (S), root (R), anther (A), petal (P), ovary (O), sepal (Se), seeds 10 days post anthesis (S1) and seeds 20 days post-anthesis (S2), were collected from plants grown in the greenhouse and analyzed. For detailed, quantitative analysis of *GUS* activity, leaf, stem and root tissues were collected from kanamycin resistant F1 progeny grown *in vitro.* Floral tissues were harvested at developmental stages 8-10 (Koltunow *et al*., 1990, *Plant Cell* **2**, 1201-1224) from the original transgenic plants. Flowers were also tagged and developing seeds were collected from capsules at 10 and 20 dpa. In all cases, tissue was weighed, immediately frozen in liquid nitrogen, and stored at -80°C.

Tissues analyzed by histological assay were at the same developmental stages as those listed above. Different hand-cut sections were analyzed for each organ. For each plant, histological assays were performed on at least two different occasions to ensure reproducibility. Except for floral organs, all tissues were assayed in phosphate buffer according to Jefferson (1987, *Plant Mol. Biol. Rep.* **5,** 387-405), with 1 mM X-Gluc (Sigma) as substrate. Flowers were assayed in the same buffer containing 20% (v/v) methanol (Kosugi *et al*., 1990, *Plant Sci.* **70,** 133-140).

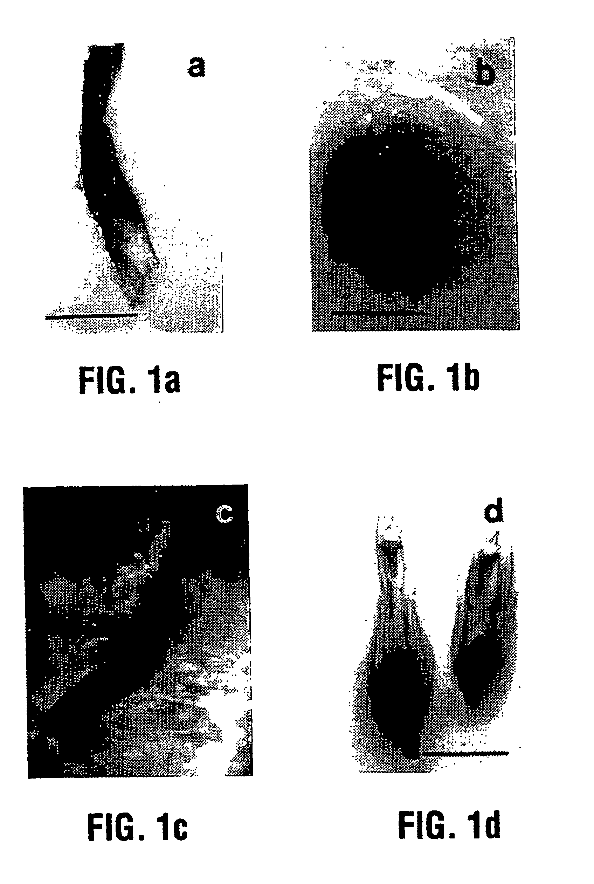

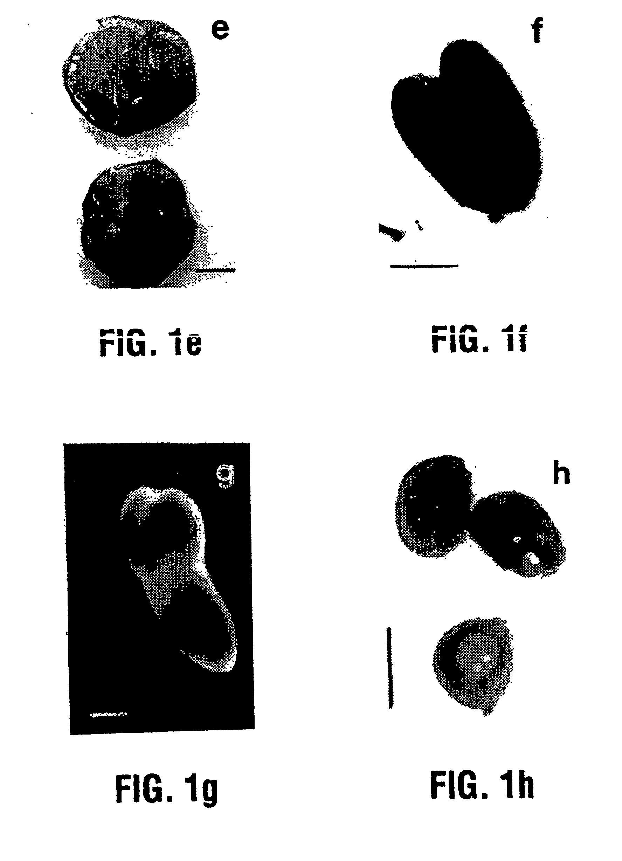

*GUS* activity in plant T1275 was found in all tissues. Figure 1 shows the constitutive expression of *GUS* by histochemical staining with X-Gluc of T1275, including leaf (a), stem (b), root (c), flower (d), ovary (e), embryos (f and g), and seed (h).

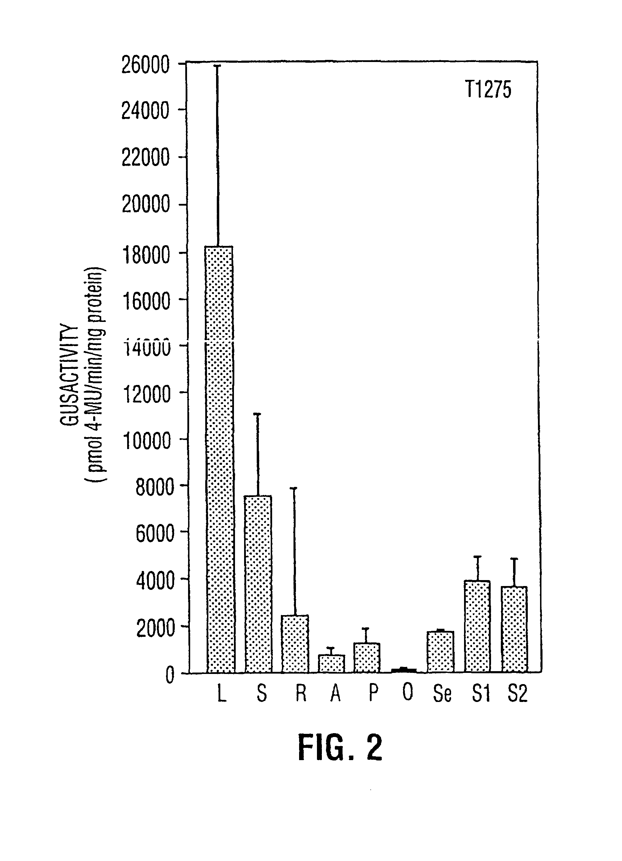

Constitutive GUS expression was confirmed with the more sensitive fluorogenic assay of plant tissue from transformed plant T1275. These results are shown in Figure 2. *GUS* expression was evident in all tissue types including leaf (L), stem (S), root (R), anther (A), pistil (P), ovary (O), sepal (Se), seeds at 10 dpa (S1) and 20 dpa (S2). Furthermore, the level of *GUS* expression is comparable to the level of expression in transformed plants containing the constitutive promoter CaMV 35S in a *GUS - nos* fusion. As reported by Fobert *et al*. (1991, Plant Molecular Biology, 17: 837-851) *GUS* activity in transformed plants containing pBI121 (Clontech), which contains a CaMV 35S - *GUS - nos* chimeric gene, was as high as 18,770 ± 2450 (pmole MU per minute per mg protein).

### Genetic Analysis of Transgenic Plant T1275

The T-DNA contains a kanamycin resistance gene. Seeds from self-pollinated transgenic plants were surface-sterilized in 70% ethanol for 1 min and in undiluted Javex bleach (6% sodium hypochloride) for 25 min. Seeds were then washed several times with sterile distilled water, dried under laminar flow, and placed in Petri dishes containing MS0 medium supplemented with 100 µg/ml kanamycin as described in Miki *et al*. (1993, Methods in Plant Molecular Biology and Biotechnology, Eds., B.R. Glick and J.E. Tompson, CRC Press, Boca Raton, 67-88). At least 90 plantlets were counted for each transformant. The number of green (kanamycin-resistant) and bleached (kanamycin-sensitive) plantlets were counted after 4-6 weeks, and analyzed using the Chi² test at a significance level of *P*<0.05.

The genetic analysis results are shown below in Table 1, which demonstrates that the T-DNA loci segregated as a single locus of insertion.

**TABLE 1**

| **Genetic Analysis of Transgenic Plant T1275** | | | | |
|---|---|---|---|---|
| No. of Progeny Km^{r} | No. of Progeny Km^{s} | Observed Ratio | Expected Ratio | Chi² |
| 262 | 88 | 3:1* | 3:1 | 0 |

| | | | | |
|---|---|---|---|---|
| * Consistent with a single dominant gene | | | | |

### Southern Blot Analysis

The T-DNA in the transgenic plant T1275 was analyzed using either a *GUS* gene coding region probe or a *nptII* gene coding region probe.

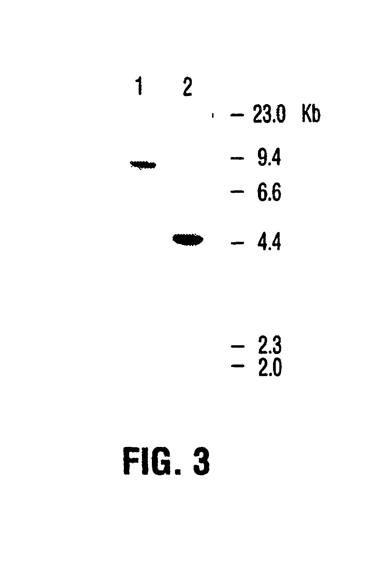

Genomic DNA was isolated from freeze-dried leaves using the protocol of Sanders *et al*. (1987, *Nucleic Acid Res.* **15,** 1543-1558). Ten micrograms of T1275 DNA was digested for several hours with *Eco*RI using the appropriate manufacturer-supplied buffer supplemented with 2.5 mM spermidine. After electrophoresis through a 0.8% TAE agarose gel, Southern blot analysis was conducted using standard protocols. As the T-DNA from the construct containing the constitutive promoter - *GUS* - *nos* construct contains only a single *Eco* RI recognition site the hybridizing fragments are composed of both T-DNA and flanking tobacco DNA sequences. The length of the fragment will vary depending on the location of the nearest *Eco* RI site. Using the *GUS* gene as a probe (Figure 3 - lane 1), the fragment to the nearest *Eco* RI site in the plant DNA will be detected. With T1275, one such fragment was located. Using the *nptII* coding region as a probe (Figure 3 - lane 2), which hybridizes to sequences on the opposite side of the *Eco* RI site, again only one hybridization band was evident. As can also be seen in Figure 3, no major rearrangements occurred within the T-DNA.

### Cloning and Analysis of the Constitutive Promoter - GUS Fusion

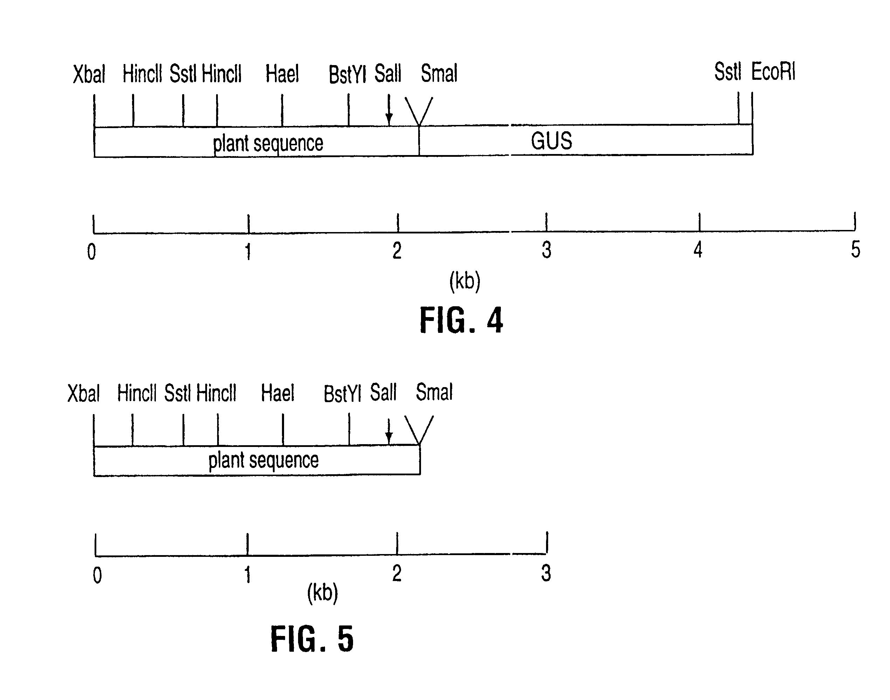

Genomic DNA was isolated from leaves according to Hattori *et al*. (1987, *Anal. Biochem.* **165,** 70-74). Ten µg of T1275 total DNA was digested with *Eco*Rl and *Xba*l according to the manufacturer's instructions. The digested DNA was size-fractionated on a 0.7% agarose gel. The DNA fragments of about 4 to 6 kb were isolated from the gel using the Elu-Quick kit (Schleicher and Schuell) and ligated to lambdaGEM-2 arms previously digested with *Eco*RI and *Xba*I and phosphatase-treated. About 40,000 plaques were transferred to a nylon membrane (Hybond, Amersham) and screened with the ³²P-labelled 2kb *GUS* insert isolated form pBI121, essentially as described in Rutledge *et al*. (1991, *Mol*. *Gen Genet.* **229,** 31-40). The positive clones were isolated. The *Xba*I-*Eco*RI fragment (see restriction map of Figure 4 and Figure 5) was isolated from the lambda phage and cloned into pTZ19R previously digested with *Xba*l and *Eco*RI and treated with intestinal calf phosphatase.

The plant DNA sequence within the clone has not been previously reported in sequence data bases. It is not observed among diverse species as Southern blots did not reveal bands hybridizing with the fragment in soybean, potato, sunflower, *Arabidopsis, B. napus, B. oleracea,* corn, wheat or black spruce (data not shown). In tobacco, Southern blots did not reveal evidence for gross rearrangements at or upstream of the T-DNA insertion site (data not shown).

For analysis of transient expression of *GUS* activity mediated by biolistics (Sandford et al, 1983, *Methods Enzymol*, 217: 483-509), the *Xbal* - *EcoRI* fragment was subcloned in pUC19 and *GUS* activity was detected by staining with X-Gluc as described above. Leaf tissue of greenhouse-grown plants or cell suspension cultures were examined for the number of blue spots that stained. As shown in Table 2, the T1275 promoter - *GUS* nos gene was active in each of the diverse species examined.

**TABLE 2**

| **Transient Expression of GUS Activity in Tissues of Diverse Plant Species** | | |
|---|---|---|
| **Tissue Source** | **Species** | ***GUS* Activity *** |
| Leaf | Soybean | +++ |
| | Alfalfa | ++ |
| | *Arabidopsis* | + |
| Leaf disc | Tobacco | ++ |
| | *B. napus* | + |
| | Pea | + |
| Cell Cultures | Oat | + |
| | Corn | + |
| | Wheat | + |
| | Barley | ++ |

| | | |
|---|---|---|
| * Numbers of blue spots: 1 - 10 (+), 10 - 100 (++), 100 - 400 (+++) | | |

The 4.2kb fragment containing about 2.2kb of the T1275 promoter fused to the *GUS* gene and the *nos* 3' was isolated by digesting pTZ-T1275 with *Hin*dIII and *Eco*RI. The isolated fragment was ligated into the pRD400 vector (Datla *et al*., 1992, *Gene*, **211**:383-384) previously digested with *Hin*dIII and *Eco*RI and treated with calf intestinal phosphatase. Transfer of the binary vector to *Agrobacterium tumefaciens* and leaf disc transformation of *N. tabacum* SR1 were performed as described above. *GUS* activity was examined in several organs of many independent transgenic lines. *GUS* mRNA was also examined in the same organ by RNase protection assay (Melton et al, 1984, *Nucleic Acids Res.* 121: 7035 - 7056) using a probe that mapped the mRNA 5' end in both untransformed and transgenic tissues. RNA was isolated from frozen-ground tissues using the TRIZOL Reagent (Life Technologies) as described by the manufacturer. For each assay 10 - 30 ug of total RNA was hybridized to an antisense RNA probe corresponding to 600 bases of the T1275 plant sequence and 400 bases of the *GUS* gene. Assays were performed using the RPAII kit (Ambion CA) as described by the manufacturer. The protected fragments were separated on a 5% Long Ranger acrylamide (J.J. Baker, N.J.) denaturing gel which was dried and exposed to Kodak X-RP film.

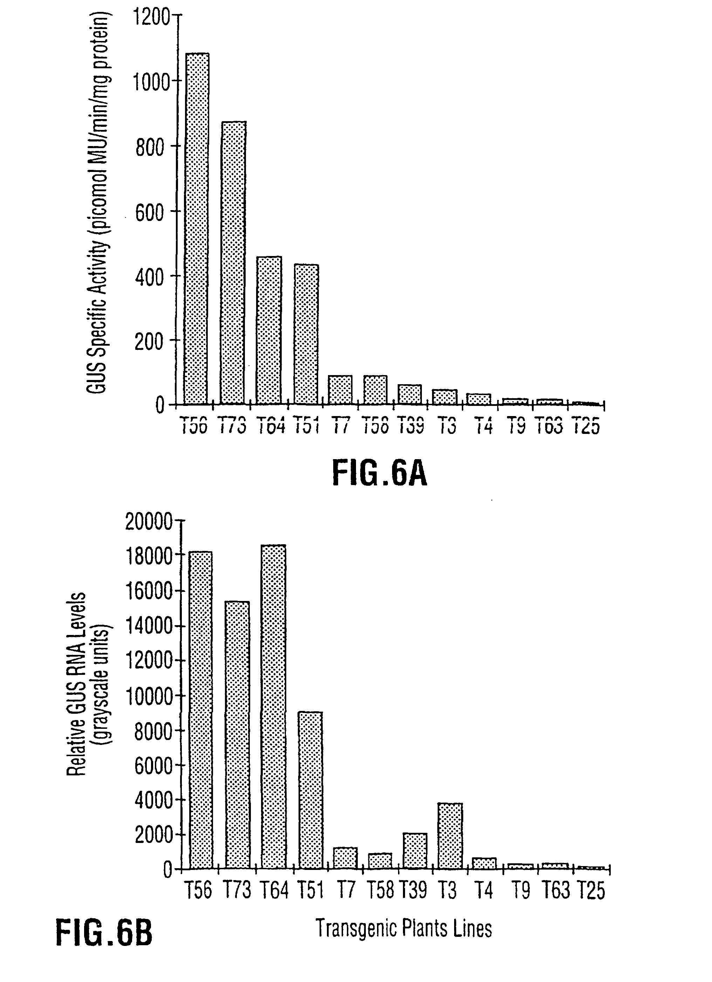

Analysis of leaves of randomly-selected, green house-grown plants regenerated from culture revealed a wide range of *GUS* specific activities (Figure 6A). Plants transformed with pBI 121 (CLONETECH) which contains the *35S*-GUS-*nos* gene yielded comparable specific activity levels (data not shown). Generally, the level of *GUS* mRNA in the leaves (Figure 6B) correlated with the *GUS* specific activities. Furthermore, the presence of a single band of about 600 bases in all samples indicated that the *GUS* transcription start site was located about 180 bases upstream of the T-DNA insertion site within the plant DNA sequence.

For analysis of *GUS* expression in different organs, lines derived from progeny of the above lines were examined in detail. Table 3 shows the *GUS* specific activities in one of these plants. It is expressed in leaf, stem, root, developing seeds and the floral organs, sepals, petals, anthers, pistils and ovaries at varying levels, confirming constitutive expression. Introduction of the same vector into *B. napus* also revealed expression of *GUS* activity in these organs (data not shown) indicating that constitutive expression was not specific to tobacco. Examination of *GUS* mRNA in the tobacco organs showed-that the transcription start sites was the same in each and the level of mRNA was similar except in flower buds where it was lower (Table 3).

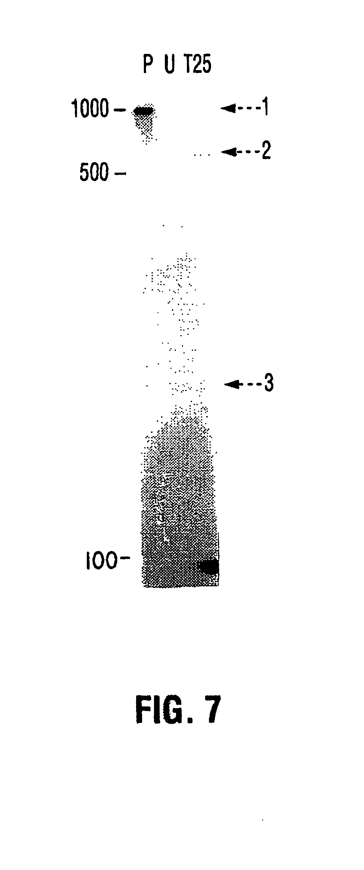

Assays performed with RNA from leaves, stem, root, developing seeds and flowers of untransformed tobacco did not reveal a protected fragment using the same probe as above (Figure 7). It is therefore assumed that the insertion site is transcriptionaly silent in untransformed tobacco and is activated by T-DNA insertion. The region upstream of the insertion site is therefore another example of a plant cryptic promoter (Fobert et al, 1994, *Plant J.* **6**:568-577).

**TABLE 3**

| *GUS* Specific Activity and Relative RNA Levels in the Organs of Progeny of Transgenic Line T64 | | | |
|---|---|---|---|
| **Organ** | **Relative *GUS* RNA Levels in T64 Progeny (grey scale units)** | ***GUS* Specific Activity (picomol/MU/min/mg protein)** | |
| | | **Transformed Tobacco T64** | **Untransformed Tobacco** |
| Leaf | 1774 | 988.32 | 3.02 |
| Stem | 1820 | 826.48 | 7.58 |
| Root | 1636 | 4078.45 | 22.18 |
| 14 day post anthesis Seeds | 1790 | 253.21 | 10.03 |
| Flower - buds | 715 | 2.59 | ND* |
| Petals | ND* | 28.24 | 1.29 |
| Anthers | ND* | 4.64 | 0.35 |
| Pistils | ND* | 9.76 | 1.72 |
| Sepals | ND* | 110.02 | 2.48 |
| Ovary | ND* | 4.42 | 2.71 |

| | | | |
|---|---|---|---|
| * Not Done | | | |

All scientific publications and patent documents are incorporated herein by reference.

The present invention has been described with regard to preferred embodiments. However, it will be obvious to persons skilled in the art that a number of variations and modifications can be made without departing from the scope of the invention as described in the following claims.

## Claims

1. An isolated promoter comprising a nucleotide sequence comprising at least an 18 bp contiguous sequence of SEQ ID NO: 1.

2. The isolated promoter of claim 1 comprising a DNA fragment as **characterized by** the restriction map of Figure 5, from Xba I to Sal I.

3. The isolated promoter of claim 1, wherein said promoter is a constitutive promoter.

4. The isolated promoter of claim 1, wherein said promoter is a cryptic promoter.

5. The isolated constitutive promoter of claim 3, wherein said promoter has a nucleotide sequence matching with at least 70%, preferably 80% and most preferably 90% of the nucleotides of SEQ ID NO: 1.

6. The isolated constitutive promoter of claim 3, wherein said promoter has a nucleotide sequence functionally equivalent to SEQ ID NO: 1, which directs the expression of an exogenous gene constitutively.

7. An isolated DNA molecule comprising the nucleotide sequence of SEQ ID NO: 1.

8. A chimeric gene construct comprising a DNA of interest for which constitutive expression is desired, and said constitutive promoter according to claim 3.

9. The chimeric gene construct according to claim 8, wherein said constitutive promoter has a nucleotide sequence matching with at least 70%, preferably 80% and most preferably 90% of the nucleotides of SEQ ID NO: 1.

10. A chimeric gene construct comprising the isolated DNA molecule of claim 7.

11. A vector comprising the chimeric gene construct of claim 9.

12. A vector comprising the chimeric gene construct of claim 10.

13. A method of conferring constitutive expression of a gene in a plant, comprising: operatively linking a DNA of interest, for which constitutive expression is desired, with a constitutive promoter from tobacco, said constitutive promoter comprising at least an 18 bp contiguous sequence of SEQ ID NO: 1, to produce a chimeric gene construct and introducing the chimeric gene construct into a plant capable of expressing the chimeric gene construct.

14. The method according to claim 13, wherein said constitutive promoter has a nucleotide sequence matching with at least 70%, preferably 80% and most preferably 90% of the nucleotides of SEQ ID NO: 1.

15. A transgenic plant cell containing the chimeric gene construct of claim 8.

16. The transgenic plant cell according to claim 15, wherein said constitutive promoter has a nucleotide sequence matching with at least 70%, preferably 80% and most preferably 90% of the nucleotides of SEQ ID NO: 1.

17. A transgenic plant cell containing the chimeric gene construct of claim 10.

18. The transgenic plant cell according to claim 16 selected from the group consisting of tobacco, *N. tabacum*, *B. napus,* soybean, alfalfa, *Arabidopsis,* corn, wheat, and barley.

19. A transgenic plant containing the chimeric gene construct of claim 8.

20. The transgenic plant according to claim 19, wherein said constitutive promoter has a nucleotide sequence matching with at least 70%, preferably 80% and most preferably 90% of the nucleotides of SEQ ID NO: 1.

21. A transgenic plant containing the chimeric gene construct of claim 10.

22. The transgenic plant according to claim 21 selected from the group consisting of tobacco, *B. napus*, soybean, alfalfa, *Arabidopsis,* com, wheat, and barley.

## Patentansprüche

1. Ein isolierter Promotor umfassend eine Nukleotid-Sequenz, welche mindestens eine 18 Basenpaar lange kontinuierliche Sequenz aus SEQ ID NO: 1 umfasst.

2. Der isolierte Promotor nach Anspruch 1, umfassend ein DNA-Fragment, wie es durch die Restriktionskarte aus Figur 5 von Xba I bis Sal I charakterisiert ist.

3. Der isolierte Promotor nach Anspruch 1, wobei der Promotor ein konstitutiver Promotor ist.

4. Der isolierte Promotor nach Anspruch 1, wobei der Promotor ein kryptischer Promotor ist.

5. Der isolierte konstitutive Promotor nach Anspruch 3, wobei dieser Promotor eine Nukleotid-Sequenz aufweist, die zu mindestens 70 %, vorzugsweise 80 % und besonders bevorzugt 90 % mit den Nukleotiden aus SEQ ID NO: 1 übereinstimmt.

6. Der isolierte konstitutive Promotor nach Anspruch 3, wobei dieser Promotor eine Nukleotid-Sequenz aufweist, die funktionell zu SEQ ID NO: 1 äquivalent ist und welche die Expression eines exogenen Gens konstitutiv steuert.

7. Ein isoliertes DNA-Molekül umfassend die Nukleotid-Sequenz von SEQ ID NO: 1.

8. Ein chimeres Gen-Konstrukt umfassend eine interessierende DNA, welche konstitutiv exprimiert werden soll und den konstitutiven Promotor nach Anspruch 3.

9. Das chimere Gen-Konstrukt nach Anspruch 8, wobei der konstitutive Promotor eine Nukleotid-Sequenz aufweist, die zu mindestens 70 %, vorzugsweise 80 % und besonders bevorzugt 90 % mit den Nukleotiden aus SEQ ID NO: 1 übereinstimmt.

10. Ein chimeres Gen-Konstrukt umfassend das isolierte DNA-Molekül nach Anspruch 7.

11. Ein Vektor umfassend das chimere Gen-Konstrukt nach Anspruch 9.

12. Ein Vektor umfassend das chimere Gen-Konstrukt nach Anspruch 10.

13. Verfahren zur Übertragung der konstitutiven Expression eines Genes in einer Pflanze, umfassend das funktionsfähige Verbinden einer interessierenden DNA, die konstitutiv exprimiert werden soll, mit einem konstitutiven Promotor aus Tabak, wobei der konstitutive Promotor eine mindestens 18 Basenpaar lange durchgängige Sequenz aus SEQ ID NO:1 umfasst, um ein chimeres Gen-Konstrukt herzustellen und das chimere Gen-Konstrukt in eine Pflanze, die fähig ist, das chimere Gen-Konstrukt zu exprimieren, einzuführen.

14. Das Verfahren nach Anspruch 13, wobei der konstitutive Promotor eine Nukleotid-Sequenz aufweist, die zu mindestens 70 %, vorzugsweise 80 % und besonders bevorzugt 90 % mit den Nukleotiden aus SEQ ID NO: 1 übereinstimmt.

15. Eine transgene Pflanze, enthaltend das chimere Gen-Konstrukt nach Anspruch 8.

16. Die transgene Pflanzenzelle nach Anspruch 15, wobei der konstitutive Promotor eine Nukleotid-Sequenz aufweist, die zu mindestens 70 %, vorzugsweise 80 % und besonders bevorzugt 90 % mit den Nukleotiden von SEQ ID NO: 1 übereinstimmt.

17. Eine transgene Pflanzenzelle, enthaltend das chimere Gen-Konstrukt nach Anspruch 10.

18. Die transgene Pflanzenzelle nach Anspruch 16, ausgewählt aus der Gruppe bestehend aus Tabak, N. Tabacum, B. Napus, Soyabohne, Alfalfa, Arabidopsis, Mais, Weizen und Gerste.

19. Eine transgene Pflanze, enthaltend das chimere Gen-Konstrukt nach Anspruch 8.

20. Die transgene Pflanze nach Anspruch 19, wobei der konstitutive Promotor eine Nukleotid-Sequenz aufweist, die zu mindestens 70 %, vorzugsweise 80 % und besonders bevorzugt 90 % mit den Nukleotiden aus SEQ ID NO: 1 übereinstimmt.

21. Eine transgene Pflanze, enthaltend das chimere Gen-Konstrukt nach Anspruch 10.

22. Die transgene Pflanze nach Anspruch 21, ausgewählt aus der Gruppe bestehend aus Tabak, B. Napus, Soyabohne, Alfalfa, Arabidopsis, Mais, Weizen und Gerste.

## Revendications

1. Promoteur isolé comprenant une séquence nucléotidique comprenant au moins une séquence de 18 pb contiguës de SEQ ID NO: 1.

2. Promoteur isolé de la revendication 1 comprenant un fragment d'ADN comme **caractérisé par** la carte de restriction de la figure 5, de XbaI à SaII.

3. Promoteur isolé de la revendication 1, dans lequel ledit promoteur est un promoteur constitutif.

4. Promoteur isolé de la revendication 1, dans lequel ledit promoteur est un promoteur cryptique.

5. Promoteur constitutif isolé de la revendication 3, dans lequel ledit promoteur a une séquence nucléotidique correspondant à au moins 70 %, de préférence 80 % et de manière préférée entre toutes 90 % des nucléotides de SEQ ID NO: 1.

6. Promoteur constitutif isolé de la revendication 3, dans lequel ledit promoteur a une séquence nucléotidique fonctionnellement équivalente à SEQ ID NO: 1, qui dirige l'expression d'un gène exogène de manière constitutive.

7. Molécule d'ADN isolée comprenant la séquence nucléotidique de SEQ ID NO: 1.

8. Construction de gène chimère comprenant un ADN d'intérêt pour laquelle une expression constitutive est recherchée, et ledit promoteur constitutif selon la revendication 3.

9. La construction de gène chimère selon la revendication 8, dans laquelle ledit promoteur constitutif a une séquence nucléotidique correspondant à au moins 70 %, de préférence 80 %, et de manière préférée entre toutes 90 % des nucléotides de SEQ ID NO: 1.

10. Construction de gène chimère comprenant la molécule d'ADN isolée de la revendication 7.

11. Vecteur comprenant la construction de gène chimère de la revendication 9.

12. Vecteur comprenant la construction de gène chimère de la revendication 10.

13. Méthode pour conférer l'expression constitutive d'un gène dans une plante, comprenant : la liaison fonctionnelle d'un ADN d'intérêt, pour lequel une expression constitutive est voulue, avec un promoteur constitutif du tabac, ledit promoteur constitutif comprenant au moins une séquence de 18 pb contiguës de SEQ ID NO: 1, pour produire une construction de gène chimère et l'introduction de la construction de gène chimère dans une plante capable d'exprimer la construction de gène chimère.

14. Méthode selon la revendication 13, dans laquelle ledit promoteur constitutif a une séquence nucléotidique correspondant à au moins 70 %, de préférence 80 % et de manière préférée entre toutes 90 % des nucléotides de SEQ ID NO: 1.

15. Cellule végétale transgénique contenant la construction de gène chimère de la revendication 8.

16. Cellule végétale transgénique selon la revendication 15, dans laquelle ledit promoteur constitutif a une séquence nucléotidique correspondant à au moins 70 %, de préférence 80 % et de manière préférée entre toutes 90 % des nucléotides de SEQ ID NO: 1.

17. Cellule végétale transgénique contenant la construction de gène chimère de la revendication 10.

18. Cellule végétale transgénique selon la revendication 16 choisie dans le groupe constitué par le tabac, *N. tabacum*, *B. napus*, le soja, la luzerne, *Arabidopsis*, le maïs, le blé et l'orge.

19. Plante transgénique contenant la construction de gène chimère de la revendication 8.

20. Plante transgénique selon la revendication 19, dans laquelle ledit promoteur constitutif a une séquence nucléotidique correspondant à au moins 70 %, de préférence 80 % et de manière préférée entre toutes 90 % des nucléotides de SEQ ID NO: 1.

21. Plante transgénique contenant la construction de gène chimère de la revendication 10.

22. Plante transgénique selon la revendication 21 choisie dans le groupe constitué par le tabac, *B. napus*, le soja, la luzerne, *Arabidopsis*, le maïs, le blé et l'orge.


### SEQUENCE LISTING

(1) GENERAL INFORMATION:
   (i) APPLICANT:
      (A) NAME: HER MAJESTY IN RIGHT OF CANADA, AS REPRESENTED BY THE MINISTER OF AGRICULTURE AND AGRI-FOOD CANADA
      (B) STREET: Central Experimental Farm
      (C) CITY: Ottawa
      (D) STATE: Ontario
      (E) COUNTRY: Canada
      (F) POSTAL CODE (ZIP): KlA 0C6

      (A) NAME: PIERRE FOBERT
      (B) STREET: 707 Tobin Terrace
      (C) CITY: Saskatoon
      (D) STATE: Saskatchewan
      (E) COUNTRY: Canada
      (F) POSTAL CODE (ZIP) : S7N 4P4

      (A) NAME: VENKATRAN N. IYER
      (B) STREET: 2595 Maquinna Road
      (C) CITY: Kelowna
      (D) STATE: British Columbia
      (E) COUNTRY: Canada
      (F) POSTAL CODE (ZIP): V1W 2R9
   (ii) TITLE OF INVENTION: Promoter From Tobacco
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 2224 base pairs
      (B) TYPE: nucleic acid
      (C) STRANDEDNESS: double
      (D) TOPOLOGY: linear
   (xi) SEQUENCE DESCRIPTION: SEQ ID NO: 1:
   TCTAGACTTA CAGAAAGTCT CTAACACGTG AGGGAATGAT CCCTTTCCTT ACCTCCCTGT 60
   AGAGATATTG GCTTTTCAAC AACTAGTACA TAAATATGCG ACTTTGACCG TGTATCCCCA 120
   GTCAAAAGGG AACTTCACCC TCCTAGTTCT TTATTTCCAA CATACATGGG GAGTAATGCT 180
   AAATTTACAT AGAAGAATAA TAAAATGAAC TGTAACTAAT GATGTACTGT TCCAAAGAGA 240
   TGAGGACGTC AACATATTTA TTCCTTCAGC CCTTTTCAGA ATAATACCAT AAGTAGAAGA 300
   AATGGCACAT AAAATGAAGT CCTCGGCAAG TCAAATGTAA ATCTGAACCC ACCCAGCTAA 360
   CCCAGTGAAC TCAACTTTCC TGGATAGATC AGCACTCCTT CATGACATTG CATGCCTTCT 420
   CTTTAAAGAG CCGCTTGATC TCTGAAAACC AAATGAATCT CCACAGAGAG ATTTCGAGCT 480
   CCATGAGACG CCTTTTGGTT CTTGATTTAC TAAACCTATA AAAATGAAAG GAAGTAGGAC 540
   AACTGCATTT TGCCGCTTAA GATGCTTCGG CGCTTTGTGA ATTTTAAGTC ATGAGAAAGT 600
   ACAATGTTGG AATCTCACAT TAGAACAATG TATTTGTAAT AACCTAGGAA AGCAAAGCTA 660
   GAAGGGAGGT GCAGCTAAAT CTTCTTCTAC CTTGTTATCC TTGCATTTCT TGAGGAGGAG 720
   GAACTGTCCT CGCAGGTGCA AAATCTGCAG TCGCCCAAAA GGATATTCAG AAGTATATTA 780
   CAACATGTTT AATGGTTAAC CAAGTGAAAG ATCAAAATAG TCATTAGAAC AAAATGCGTG 840
   CTCAGAGCGT ATCTACTAGT TCATCAACCC AGTACACATC TCTGAATTTC ATCTCTTGCC 900
   GTTGAACTAA GTCAATTGGT CAAAGACGCA TAACATGAGA GACACTCATA AAACGGCTGA 960
   ATAACATGCA GAAGACGTCA TGCGCCTTAG GTCTCATTAT GCATGAGATT ATTAGTTATA 1020
   TGCTCCTTCA GTTTGACTAG AAATGAAAAA TCAGTTAAGC CTGTAACGAA ATGATAACCT 1080
   GCTTCAAGAA GATTAGACTA TTTTTCATAA AATATGCAGT GCCGTGAAAT AGATACTTAA 1140
   TCTTAGGCAG GAAAAATCTT CTATTGGGCC ATAATAAGAA CTACCAATTA GAAAGGAGGT 1200
   AGAAAGCTCC GATACTGTTA TGAAGGCCAT TCTAAGTGCT GATGTGAATT TCCCAATACA 1260
   AAATGACAAC AAAAACAAAA GCCTCAATCC TAAGCTAGTT GGGGTCGCTA TATAAATCCT 1320
   CGACATCCAT TTAACTCCAC TTGGACTCCT TTCTTTCCAA TATTTTAATA TTGTTAGATT 1380
   AATCATAAAA TTGCTTAGCT TTCTACTGGC ACTTAACCTA CTGCAACCCT CCTCTTCTGG 1440
   GATTCCAACA CAAACAACTA AGAGGAATTT GAAAAAAAGA AAGCAAATGT GAGAAGAGAC 1500
   AAAATGTACA ATGATACCTC TTCTTGCAGC AAAGGAGGCA GGTTCTCTGC TGAGACAAGG 1560
   TTCTCTATTT CCTGCAAGAC CTTCGTATCT TTTATTCGAG ACCATGTATG TGGAGGTAAC 1620
   GCCAGCAATA GTGCTGTCAG CACATCGTTG CTTGCAGGGG ATCTTCTGCA AGCATCTCTA 1680
   TTTCCTGAAG GTCTAACCTC GAAGATTTAA GATTTAATTA CGTTTATAAT TACAAAATTG   1740
   ATTCTAGTAT CTTTAATTTA ATGCTTATAC ATTATTAATT AATTTAGTAC TTTCAATTTG   1800
   TTTTCAGAAA TTATTTTACT ATTTTTTATA AAATAAAAGG GAGAAAATGG CTATTTAAAT   1860
   ACTAGCCTAT TTTATTTCAA TTTTAGCTTA AAATCAGCCC CAATTAGCCC CAATTTCAAA   1920
   TTCAAATGGT CCAGCCCAAT TCCTAAATAA CCCACCCCTA ACCCGCCCGG TTTCCCCTTT   1980
   TGATCCAGGC CGTTGATCAT TTTGATCAAC GCCCAGAATT TCCCCTTTTC CTTTTTTAAT   2040
   TCCCAAACAC CCCTAACTCT ATCCCATTTC TCACCAACCG CCACATATGA ATCCTCTTAT   2100
   CTCTCAAACT CTCTCGAACC TTCCCCTAAC CCTAGCAGCC TCTCATCATC CTCACCTCAA   2160
   AACCCACCGG AATACATGGC TTCTCAAGCC GTGGAAACCT TATACTCACC TCCCTTTGCT   2220
   CTTA                                                                2224